# EUROPEAN PATENT APPLICATION

(11) **EP 2 682 477 A1**
(43) Date of publication of application: **08.01.2014**
(21) Application number: 12175354.5
(22) Date of filing: 06.07.2012
(51) Int. Cl.: C12Q 1/68

(54) **miR-181a and miR652 as oncologic biomarker of breast cancer**

(71) Applicant: National University of Ireland, Galway, Galway (IE)
(72) Inventor: Kerin, Michael J., Galway (IE); Miller, Nicola, Galway (IE); McDermott, Ailbhe, Galway (IE)
(74) Representative: Gates, Marie Christina Esther

(57) **Abstract**

The invention provides methods of and diagnostic kits for the detection of breast cancer or to assist in assessing the prognosis, or determine the efficacy of a treatment regimen for breast cancer comprising at least one oligonucleotide probe capable of binding to at least a portion of a circulating miRNA selected from the group comprising the *miR-181a* and *miR-652* biomarkers. The invention also provides methods of identifying a therapeutic agent capable of preventing or treating cancers, including breast cancer, comprising testing the ability of the potential therapeutic agent to enhance the expression of at least one protein selected from the group comprising *miR-181a* and *miR-652.*

## Description

### Field of the Invention

The present invention relates to the diagnosis of breast cancers, in determining the prognosis of a breast cancer patient and prediction of a response to cancer treatment. The goal of the invention is to allow early detection and diagnosis of breast cancers as well as individualised treatment for patients. The markers are particularly suitable for the detection and assessment of Luminal A breast cancer.

### Background to the Invention

Breast cancer is an extremely important disease in Irish Society. It is the most commonly diagnosed malignancy in Irish women (with over 2,600 new diagnoses annually), and accounts for the greatest number of cancer related deaths in women. With 1 in 8 Irish women being diagnosed with breast cancer, it is a disease which impacts, in some way, most peoples lives. Breast cancer is an extremely prevalent disease accounting for the greatest number of cancers diagnosed and the greatest number of deaths from cancer in women in Ireland. When diagnosed and treated early, breast cancer is a highly curable disease, and the past decade has witnessed major advances in its management. However, many women continue to die from this disease process as a consequence of late diagnosis, with incurable metastases at the time of presentation. The focus of the invention is on the development of sensitive, specific, minimally invasive biomarkers for breast cancer that can be utilised to detect early tumours, as well as being applied to monitoring the response to treatment, and detecting disease recurrence. MiRNAs are a class of small, non-coding RNA fragments that have captured the attention and innovation of the scientific community since their discovery almost twenty years ago. The discovery that MiRNAs are dysregulated in several disease processes, including carcinogenesis, has unveiled their putative role as disease-specific biomarkers and therapeutic targets.

Currently diagnosis of breast cancer involves a combination of clinical examination, radiological imaging and an invasive tissue biopsy, to provide histological confirmation. Mammography is currently considered the gold standard for diagnosis, yet it is not without its constraints, with both ionisation exposure and a false positive rate of up to 10%. Small curable early cancers can potentially be missed. When diagnosed promptly, women are more likely to have early stage disease, confined to the breast. These cases are amenable to breast conserving surgery. In cases where tumour cells have metastasised to the lymph nodes at the time of diagnosis more extensive local surgery and auxiliary surgery (an auxiliary lymph node clearance is often required). This procedure carries the potential for severe complications, including lymphoedema, which impacts extensively on one's quality of life. More over, tumour cells in the lymph nodes may be an indicator of distant metastases elsewhere, that are undetectable by current strategies. Current practice demands that the majority of these women receive adjunct chemotherapy and/or hormonal therapies. The decision to commence such regiments should not be taken lightly as each of these treatment modalities has an associate cluster of adverse effects. The appropriateness of adjunct therapy is usually based on a culmination of histological and patient factors. Only two predicted markers are validated and routinely assessed in the management of breast cancer. One of these is Oestrogen Receptor (ER) status and the other is Her2/neureceptor status. These markers indicate that there is a likely benefit to be achieved from treatment with hormonal therapies or Trastuzumab, respectively.

MicroRNAs are a class of small non-coding RNA fragments that are ideal biomarker candidates and therapeutic targets. MiRNAs have been demonstrated to play a key role in practically all aspects of the cell cycle. They function at a post-transcriptional level to cause either transcriptional cleavage or transcriptional repression. MiRNAs are abberently expressed in almost all pathological conditions, including carcinogenesis and have a putative role as oncogenes or tumour suppressor genes. MiRNAs have the advantage that they can be detected in the systemic circulation, and thus diagnostic assay is based on miRNAs are not invasive.

The concept of breast screening is now accepted internationally and is associated with an improved outcome from breast cancer due to its earlier diagnosis. However, it is not without its drawbacks; a high proportion of lobular carcinomas are mammographically occult dense breasts make the interpretation of the mammogram difficult and there are high false negative rates. Therefore, the development of a blood based diagnostic tool would be a significant advantage as it would permit screening to begin at a younger age and could potentially successfully diagnose tumours which are not detectable by mammography.

Over the past decade our understanding of breast cancer has advanced remarkably. It is a heterogeneous disease, with distinct molecular subtypes (Luminal A, Luminal B, HER2/*neu* over-expressing and Basal). Luminal A is the most common sub-type of breast cancer with over 70% of breast tumours falling into this category. Luminal A tumours are characterised by hormone receptor positivity (oestrogen receptor and progesterone receptor) and HER2/*neu* negativity. Women diagnosed with Luminal A tumours typically have a good prognosis, with a five year survival over 90%. However, with most breast tumours being Luminal A, it forms a good starting point on which to base a diagnostic test.

### Object of the Invention

It is an objective of the present invention to identify novel biomarkers which could be used in the diagnosis, prognosis or prediction of response to medical treatment of breast cancer. It is a further objective to identify a minimally invasive marker for use as an adjunct in breast cancer diagnosis and/or assessment of response to treatment.

### Summary of the Invention

According to the present invention there is provided a diagnostic kit for the detection of breast cancer or to assist an assessment of prognosis, or to determine the efficacy of a treatment regimen for breast cancer, comprising at least one oligonucleotide probe capable of binding to at least a portion of a circulating miRNA selected from the group comprising *miR-181a* and *miR-652* biomarkers. The kit may comprise an oligonucleotide probe capable of binding to at least a portion of a circulating *miR-181a* and an oligonucleotide probe capable of binding to at least a portion of the circulating *miR-652.*

The kit may be adapted for performance of an assay selected from a real-time PCR assay, a micro-array assay, histochemistry assay or an immunological assay.

The kit is particularly suited for use in Luminal A breast cancer detection or assessment. The invention also provides a method of identifying a therapeutic agent capable of preventing or treating cancers, including breast cancer, comprising testing the ability of the potential therapeutic agent to enhance the expression of at least one circulating miRNA selected from the group comprising *miR-181a* and *miR-652.*

In a still further aspect the invention provides for use of a circulating miRNA selected from *miR-181a* and *miR-652* to detect breast cancer, to stratify patients according to expected prognosis or to assess the efficacy of a medical treatment.

In this use the detection may be carried out in a blood sample or a sample derived from blood.

The invention also provides a method of detecting or screening for early stage breast cancers comprising analysing a sample of blood taken from a patient for the presence of one or more biomarkers selected from the group comprising *miR-181a* and *miR-652,* the presence of at least one of these miRNAs in the sample indicating the presence of breast cancer.

### Brief Description of the Drawings

Figure 1 - The validation cohort of the study. A group of forty-seven cancer patients were compared with a control group of forty-seven normal subjects.
Figure 2 - Circulating miRNA Expression level showing there was no significant difference in expression levels between the two groups in 5 of the 7 miRNAs studied.
Figure 3 - Circulating *miR-181a* Expression showing *miR-181a* was significantly down-regulated in the blood of women with breast cancer compared to those who do not have cancer.
Figure 4 - Circulating *miR-652* Expression showing *miR-652* was significantly down-regulated in the blood of women with breast cancer compared to those who do not have cancer.
Figure 5 -Circulating *miR-181a* and *miR-652* Expression level Stage of Disease - An important trait in any biomarker is the ability to detect both early and late stage disease, both miRNAs were down-regulated in early and late stage disease.
Figure 6 - Correlation between miR-*181a* level and Invasive Tumour Size
Figure 7 - Binary logistics regression analysis of a combination of *miR-181a* and *miR-652.* Area under the curve is AUC 0.77 with a Sensitivity of 70% and a Specificity of 65%.
Figure 8 - The two novel biomarkers of the present invention, in combination, provide a sensitivity and specificity profile which exceeds that of several current clinically used biomarkers.

### Detailed Description of the Invention

Seven miRNAs were evaluated for their utility as oncological biomarkers. These candidate markers were *miR-181a, miR-301a, miR-182, miR-423-5p, miR-93, miR-652* and *miR-19b.* Ethical approval was obtained and written informed consent was obtained from all study participants in advance of blood sampling. Women with breast cancer were identified at the Symptomatic Breast Unit where they were referred by their General Practitioner for investigation of a newly palpable breast lesion, or other symptoms suggestive of malignancy. All women in the cancer group had Luminal A breast cancer which was confirmed by histology, immunohistochemistry and fluorescence *in situ* hybridization (FISH). Demographic details and clinicopathological parameters were prospectively collected. Whole blood samples were prospectively collected from women with a new diagnosis of Luminal A breast cancer, at the time of diagnosis. Whole blood samples were also collected from healthy female volunteers who served as the control group. Venous non-fasting whole blood was collected in BD Vacutainer K2E blood bottles containing 18mg EDTA as an anticoagulant (BD-Plymouth), immediately transferred to the surgical laboratory and stored at minus 4°C until required.

Total RNA was extracted from 1ml of room temperature whole blood using TRI Reagent BD technique (Molecular Research Centre, Inc). RNA concentration and integrity were analyzed respectively by conducting NanoDrop Spectrophotometry to evaluate the 260/280 ratio (NanoDrop ND-1000 Technologies Inc., DE, USA) and using the Agilent Bioanalyzer (Agilent Technologies, Germany).

Taqman miRNA assays were purchased from ABI (Applied Biosystems, Foster City, CA, USA) for each of the 7 miRNAs: *miR-19b, miR-93, miR-181a, miR-182, miR-301a, miR-423-5p* and *miR-652.* Each RNA sample was reverse transcribed for each specific miRNA target primer, and real time quantitative polymerase chain reaction (RT-qPCR) was then conducted using the appropriate TaqMan miRNA primers and probes, as described by the manufacturer. In brief, 100ng of total RNA was reverse transcribed using MultiScribe, followed by RT-qPCR on a 7900 HR Fast Real-Time PCR System (Applied Biosystems). RT-qPCR was performed in triplicate for each target miRNA, and for each RNA sample. Inter-assay controls and intra-assay controls were used throughout. The cycle thresholds (Ct) for each sample in this cohort were normalized to *miR-16,* a validated endogenous control for use in breast cancer. MiRNA expression levels were determined using QBase Plus software.

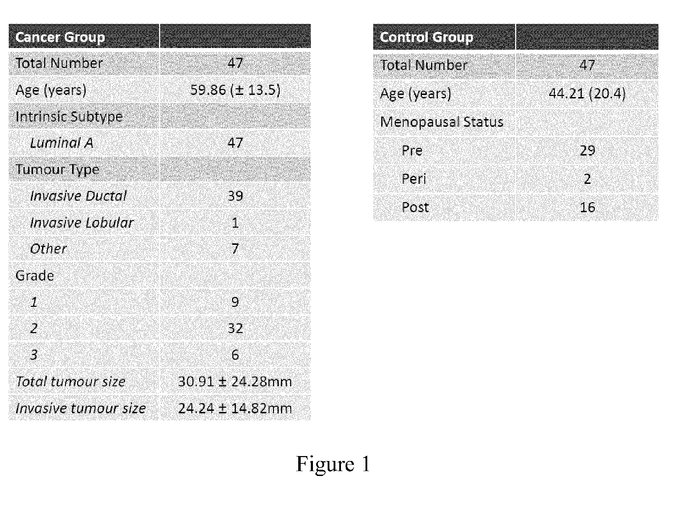

A group of forty-seven cancer patients were compared with a control group of forty-seven normal subjects. The validation cohort is shown in Figure 1.

### Validation Study Statistical Analysis

The data generated from the validation phase was analysed using Minitab 16.0 for Windows. A log transformation of the miRNA expression level was performed, given the wide range of expression values. The t-test and ANOVA were used to compare miRNA expression levels between groups. Results with a p<0.05 were considered to be statistically significant. The expression profiles *of miR-181a* and *miR-652* were used to generate a Receiver Operating Characteristic (ROC) curve.

### Results

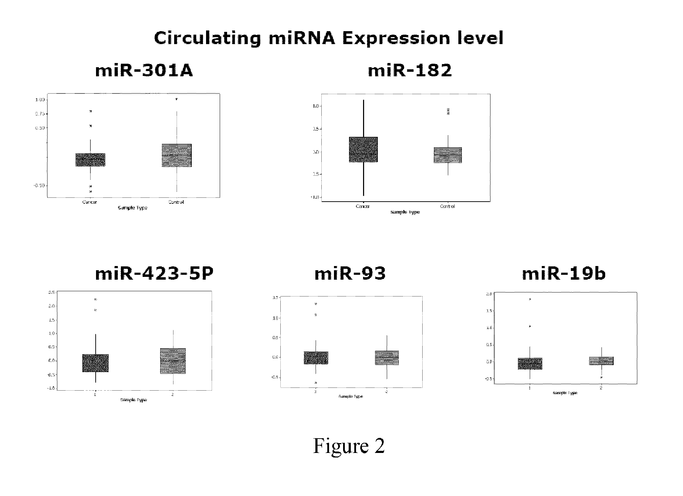

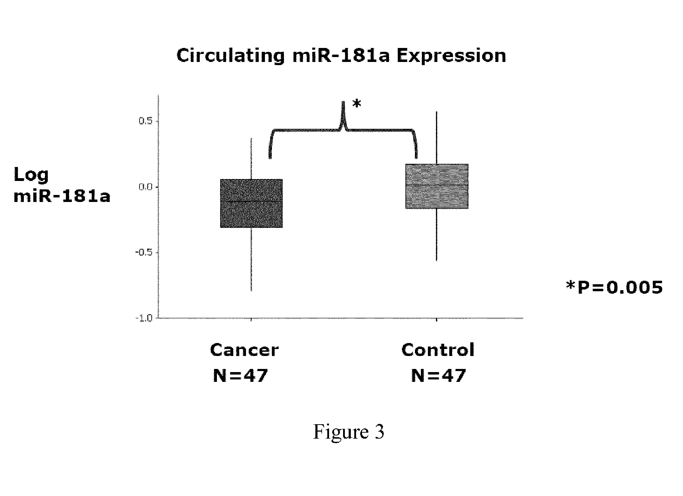

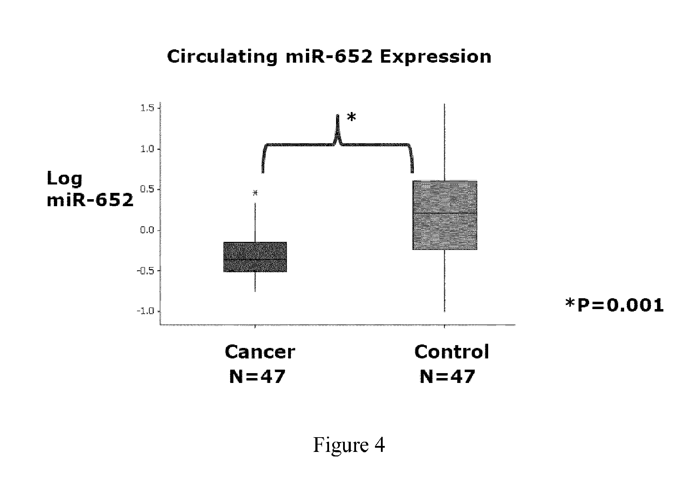

Circulating miRNA expression level was determined as outlined above for 47 Luminal A samples and 47 control samples. The patient demographic details are summarised in Figure 1. There was no significant difference in expression levels between the two groups in five of the seven miRNAs; namely *miR-301a miR-182, miR-423-5p, miR-93* and *miR-19b,* as shown in Figure 2. However, as shown in Figure 3, circulating *miR-181a* was significantly down-regulated in the blood of women with breast cancer, compared to those who did not have cancer (p=0.005). Similarly, as shown in Figure 4, *miR-652* was significantly down-regulated in the blood of women with breast cancer compared to those normal patients (p=0.001).

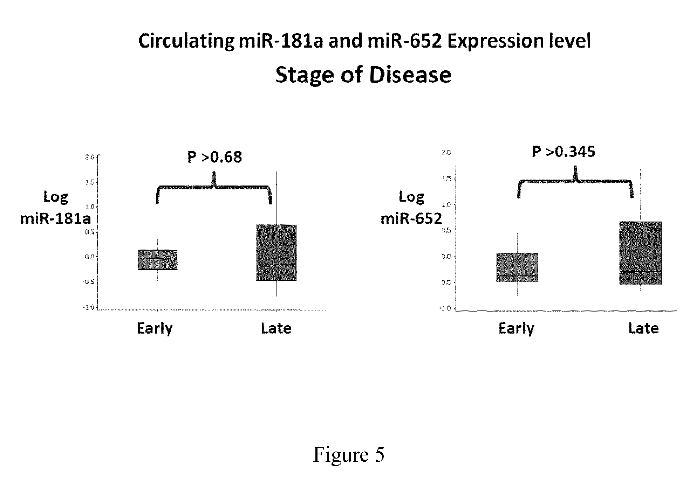

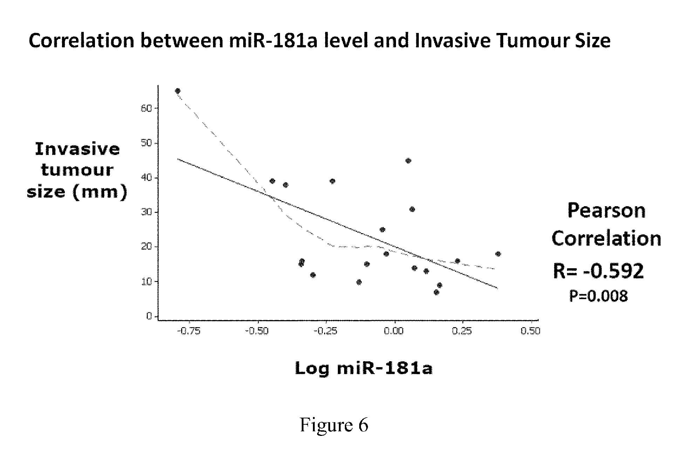

It is an important attribute of any biomarker that it is able to detect both early and late stage disease. As shown in figure 5, both *miR-181a* and *miR-652* were down-regulated in both early and late stage disease. Figure 6 also shows that there is a correlation between the circulating expression level of *miR-181a* and invasive tumour size.

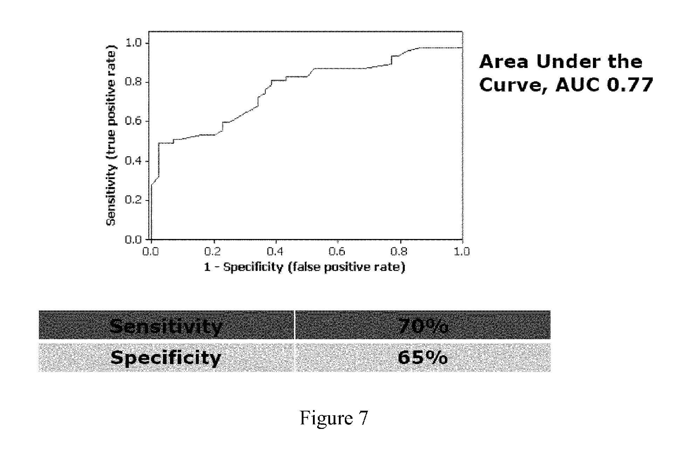

Binary logistics regression analysis was performed in order to determine the accuracy of a combination of both *miR-181a* and *miR-652* in predicting women with Luminal A breast cancer from controls, as shown in Figure 7. The area under the curve (AUC) was 0.77, which models a combination of the sensitivity and specificity. The sensitivity and specificity of the combination of *miR-181a* and *miR-652* for distinguishing between those with cancer and the controls was 70% and 65%, respectively.

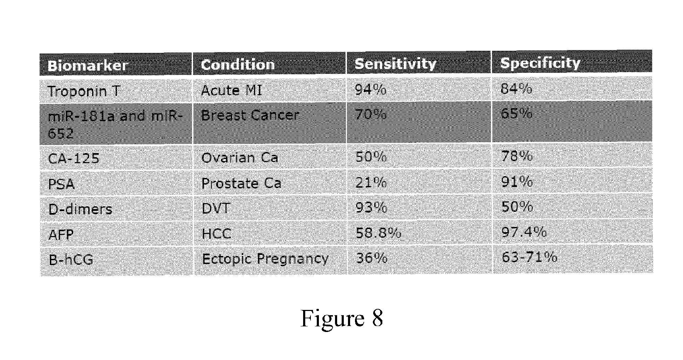

As shown in Figure 8, the two novel biomarkers identified in the present application, in combination, provide a sensitivity and specificity profile which exceeds that of several current clinical biomarkers.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A diagnostic kit for the detection of breast cancer or to assist prognosis, or determine the efficacy of a treatment regime for breast cancer comprising at least one oligonucleotide probe capable of binding to at least a portion of a circulating miRNA selected from the group comprising *miR-181a* and *miR-652* biomarkers.

2. A kit as claimed in Claim 1, comprising an oligonucleotide probe capable of binding to at least a portion of a circulating *miRNA-181a* and an oligonucleotide probe capable of binding to at least a portion of the circulating *miR-652.*

3. A kit as claimed in any preceding claim adapted for performance of an assay selected from a real-time PCR assay, a micro-array assay, histochemical assay or an immunological assay.

4. A kit as claimed in any preceding claim for use in detecting Luminal A breast cancer.

5. A method of identifying a therapeutic agent capable of preventing or treating cancers, including breast cancer, comprising testing the ability of the potential therapeutic agent to enhance the expression of at least one circulating miRNA selected from the group comprising *miR-181a* and *miR-652.*

6. Use of a circulating miRNA selected from *miR-181a* and *miR-652* to detect breast cancer, to stratify patients according to expected prognosis or to assess the efficacy of a medical treatment.

7. Use as claimed in Claim 6, wherein the detection is carried out in a blood sample or a sample derived from blood.

8. A method of detecting or screening for early stage breast cancers comprising analysing a sample of blood taken from a patient for the presence of one or more biomarkers selected from the group comprising *miR-181a* and *miR-652,* the presence of at least one of these miRNAs in the sample indicating the presence of breast cancer.
